# EUROPEAN PATENT APPLICATION

(11) **EP 2 623 147 A1**
(43) Date of publication of application: **07.08.2013**
(21) Application number: 11828722.6
(22) Date of filing: 06.09.2011
(51) Int. Cl.: A61M 5/34, A61M 5/28, A61M 5/32

(54) **MEDICAL INSTRUMENT WITH ATTACHED NEEDLE**

(30) Priority: 27.09.2010 JP 2010215362
(71) Applicant: Terumo Kabushiki Kaisha, Tokyo 151-0072 (JP)
(72) Inventor: TACHIKAWA Kouichi, Fujinomiya-shi Shizuoka (JP); OGAWA Junichi, Nakakoma-gun Yamanashi 409-3853 (JP)
(74) Representative: TBK
(86) International application number: PCT/JP2011/070210
(87) International publication number: WO 2012/043161

(57) **Abstract**

A medical instrument with attached needle includes a needle tube (2) having a needle tip (2a) capable of piercing a living body and a needle holding member (5) for fixing and holding a middle portion of the needle tube (2) by high frequency welding so that the needle tip (2) protrudes from one end and a proximal portion (2b) of the needle tube (2) protrudes from the other end. It also has a seal member (8) composed of an elastic member and provided on an outer circumference of the proximal portion (2b) of the needle tube (2) so as to be liquid-tight, and a liquid containing member (9) having a liquid chamber capable of containing a liquid therein. With the proximal portion (2b) of the needle tube (2) communicating with the liquid chamber, the seal member (8) is compressed by and clamped between the needle holding member (5) and the liquid containing member (9).

## Description

### Technical Field

The present invention relates to a medical instrument with attached needle in which a needle is preliminarily attached to a needle holding member.

### Background Art

In recent years, in view of a problem that glass-made syringes would suffer cracking in a distribution process thereof, plastic-made syringes have come to be used more frequently all over the world.

Conventionally, fixing of a needle to a plastic-made syringe has been carried out mainly by use of an adhesive. In a prefilled syringe in which a syringe is preliminarily filled with a drug, however, such an adhesive may come into contact (liquid contact) with the drug in the syringe, possibly producing a bad influence on the drug. In view of this, in prefilled syringes such as the one described in Patent Document 1, a configuration is generally adopted in which a needle is attached, by high frequency welding, to a syringe or to a needle holding member (needle hub) attached to the tip of the syringe.

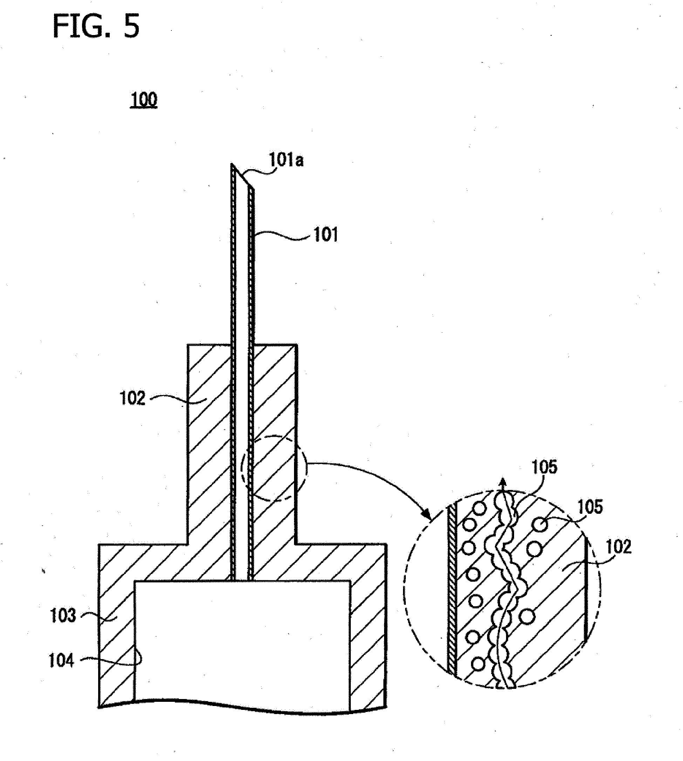

FIG. 5 shows, as an example of the related art, a schematic sectional configuration view and an enlarged view of a major part thereof, illustrating a case where a needle is attached to a tip of a syringe by high frequency welding. As shown in FIG. 5, in the conventional syringe 100, a tubular liquid containing member 103 having a liquid chamber 104 therein is provided at a tip portion (distal end portion) thereof with a cylindrical needle holding section 102 formed to have a diameter smaller than the outside diameter of the liquid containing member 103. In the needle holding section 102, a needle 101 is fixed by the high frequency welding so that a needle tip 101a formed at one end of the needle 101 protrudes from an end face of the needle holding member 102 and that the other end is communicating with the liquid chamber 104.

Meanwhile, in the case of fixing the needle 101 to the needle holding section 102 by the high frequency welding, a plurality of bubbles 105 are generated in the needle holding section 102, as shown in the enlarged view in FIG. 5. In the case where the bubbles 105 are individually independent bubbles, they do not matter. In the case where the plurality of such bubbles 105 are formed continuously, however, a part where the continuous bubbles 105 are present becomes brittle, and a crack may be generated in the direction of an arrow, as shown in FIG. 5. When the crack is thus generated in the needle holding section 102, the drug contained in the liquid containing member 103 may leak to the exterior. In the high frequency welding, it is difficult to perform such a control that the bubbles 105 generated will be independent, and it is difficult to prevent formation of the continuous bubbles.

### Prior Art Document

### Patent Document

Patent Document 1: Japanese Patent Laid-open No. Hei 08-117334

### Summary of Invention

### Technical Problem

Accordingly, as a method for fixing a needle to a plastic-made syringe, a new fixing method is needed wherein no bad influence is produced on a drug contained in a syringe and wherein liquid leakage would not occur. Besides, problems of cracking and liquid leakage upon high frequency welding of a needle may occur generally in all of medical instruments with attached needles in which the needle is fixed to a plastic-made needle holding section or member, such as a medical instrument with attached needle which is put to use in the state of being attached to a tip of a tube permitting blood or a medical agent to flow therethrough.

In consideration of the above-mentioned points, it is an object of the present invention to provide a medical instrument with attached needle in which generation of leakage of liquid to the exterior is restrained and enhanced reliability is thereby promised.

### Technical Solution

In order to solve the above-mentioned problems and attain the object of the present invention, there is provided according to the present invention a medical instrument with attached needle which includes a needle tube, a needle holding member, a seal member, and a liquid containing member. The needle tube has a needle tip capable of piercing (puncturing) a living body. The needle holding member fixes and holds a middle portion of the needle tube by high frequency welding in a condition where the needle tip protrudes from one end of the needle holding member and a proximal portion (base end portion)
on a side opposite to the needle tip of the needle tube protrudes from the other end of the needle holding member. The seal member is composed of an elastic member, and is provided on an outer circumference on a side of the proximal portion of the needle tube in a liquid-tight manner. The liquid containing member has a liquid chamber capable of containing a liquid therein. The seal member is compressed by and clamped between the needle holding member and the liquid containing member in a condition where the proximal portion of the needle tube and the liquid chamber communicate with each other.

In the medical instrument with attached needle according to the present invention, fixing of the needle tube is conducted by the high frequency welding of the needle tube to the needle holding member. In addition, fixing of the needle tube and the liquid containing member to each other is made in the liquid-tight manner by the seal member. Since the liquid containing member and the needle holding member are formed separately, it is ensured that even if the needle holding member is cracked, no influence would be produced on the liquid containing member. Consequently, generation of leakage of liquid is restrained.

### Advantageous Effect

According to the present invention, the seal member composed of the elastic member ensures that the liquid-tightness between the needle holding member and the needle tube is maintained, and generation of leakage of liquid to the exterior is restrained. In addition, since no adhesive comes into contact with the liquid contained in the liquid containing member, enhanced reliability is promised.

### Brief Description of Drawings

[FIG. 1]

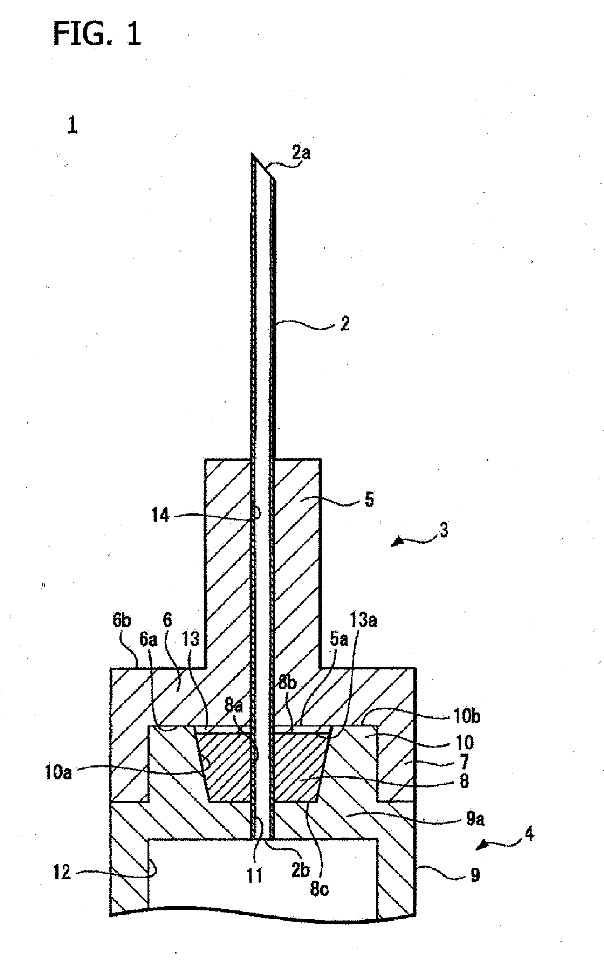

   FIG. 1 is a sectional view showing a configuration of a medical instrument with attached needle according to a first embodiment of the present invention.
[FIG. 2]

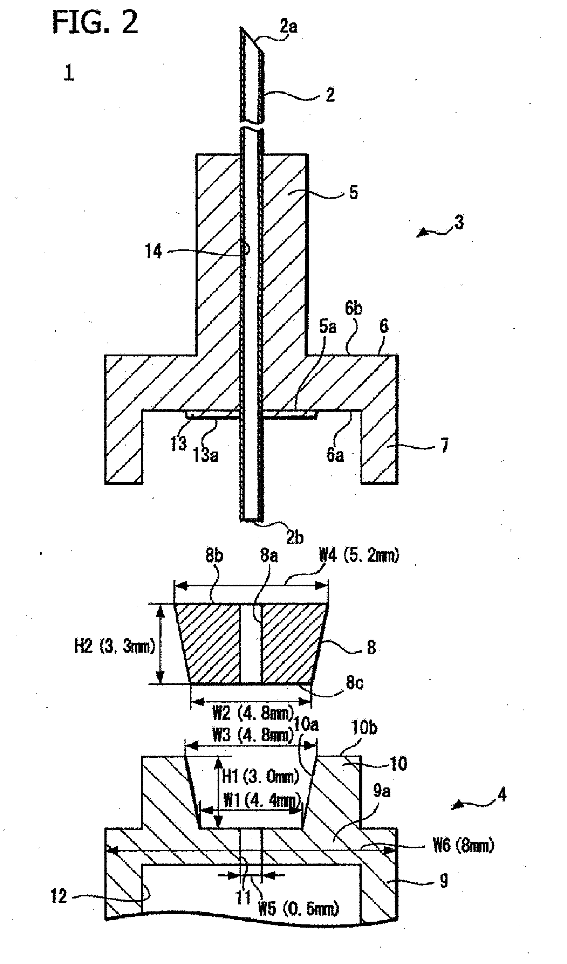

   FIG. 2 is a sectional view showing a pre-assembly state of the medical instrument with attached needle according to the first embodiment of the present invention.
[FIG. 3]

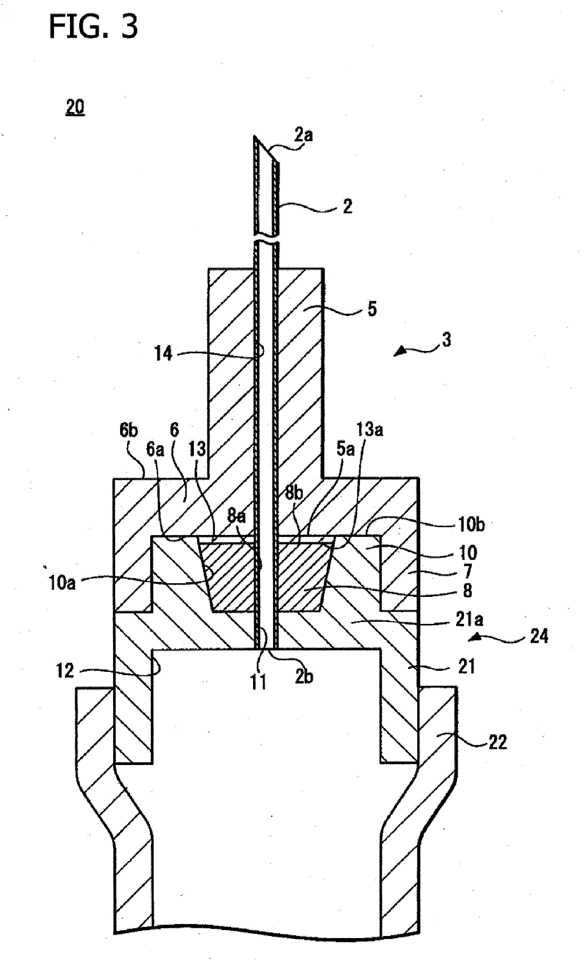

   FIG. 3 is a sectional view showing a configuration of a medical instrument with attached needle according to a modification of the first embodiment of the present invention.
[FIG. 4]

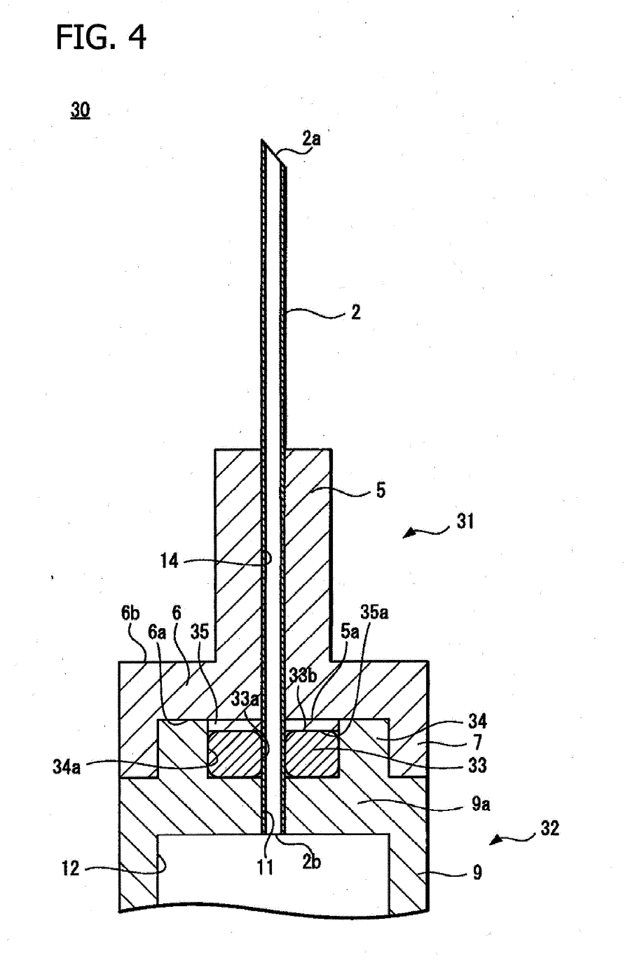

   FIG. 4 is a sectional view showing a configuration of a medical instrument with attached needle according to a second embodiment of the present invention.
[FIG. 5]
   FIG. 5 shows a sectional view, and an enlarged view of a major part thereof, illustrating a configuration of a medical instrument with attached needle according to an example of the related art.

### Mode for Carrying Out the Invention

Now, some embodiments (exemplary modes) of a medical instrument with attached needle according to the present invention will be described below, referring to FIGS. 1 to 4. Incidentally, members shown in common in the drawings are denoted by the same reference symbols. Besides, the present invention is not limited to the following embodiments (modes).
Incidentally, description will be made in the following order.
1. First Embodiment: Medical Instrument with Attached Needle
1-1. Configuration Example of Medical Instrument with Attached Needle
1-2. Method for Assembly of Medical Instrument with Attached Needle
1-3. Modification
2. Second Embodiment: Medical Instrument with Attached Needle

### <1. First Embodiment: Medical Instrument with Attached Needle>

FIG. 1 is a sectional view showing a configuration of a medical instrument with attached needle 1 according to a first embodiment of the present invention, and FIG. 2 is a sectional view showing a pre-assembly state of the medical instrument with attached needle 1. In this embodiment, a prefilled syringe which is preliminarily filled with a liquid medicine will be described as the medical instrument with attached needle 1.

### [1-1. Configuration Example of Medical Instrument with Attached Needle]

As shown in FIG. 1, the medical instrument with attached needle 1 according to this embodiment includes a needle tube 2, a needle holding member 3 for holding the needle tube 2, a seal member 8, and a liquid containing member 4 having a liquid chamber 12 capable of containing a drug therein.

As the needle tube 2, a needle tube with a size (outside diameter: 3.4 to 0.1 mm) of 10 to 36 gauge, preferably a size (outside diameter: 1.6 to 0.2 mm) of 14 to 33 gauge, according to the ISO medical needle tube standard (ISO9626: 1991/Amd. 1:2001(E)) is used. The needle tube 2 is provided at one end thereof with a needle tip 2a having a cutting edge surface 2A. The axial-directionally other end, on the side opposite to the needle tip 2a, of the needle tube 2 will be hereinafter referred to as a proximal portion 2b.

A material of the needle tube 2 may be, for example, stainless steel; however, this is not restrictive, and other metals such as aluminum, aluminum alloys, titanium, and titanium alloys can also be used as the material. In addition, the needle tube 2 is not limited to a straight needle, and may be a tapered needle which is tapered at least at a part thereof. It suffices for the tapered needle to have a proximal portion greater in diameter than a needle tip portion and to have a middle portion of a tapered structure. Besides, the sectional shape of the needle tube 2 is not restricted to a circle but may be a polygon such as a triangle.

The needle holding member 3 includes a needle holding member main body 5, a flange section 6, a fitting section 7, and a projected section 13, which are formed integrally. Examples of a material of the needle holding member 3 include various resins such as polyvinyl chloride, polyethylene, polypropylene, cyclic polyolefins, polystyrene, poly(4-methylpentene-1), polycarbonates, acrylic resins, an acrylonitrile-butadiene-styrene copolymer, polyesters such as polyethylene terephthalate, etc., a butadiene-styrene copolymer, and polyamides (e.g., nylon 6, nylon 6·6, nylon 6·10, nylon 12). Among these, preferred are such resins as polypropylene, cyclic polyolefins, polyesters, and poly(4-methylpentene-1) from the point of view of easy molding.

The needle holding member main body 5 is formed in a cylindrical shape. It fixes by enveloping a middle portion of the needle tube 2 in the condition wherein the needle tip 2a capable of piercing a living body protrudes from one end thereof and the proximal portion 2b on the side opposite to the needle tip 2a protrudes from the other end thereof. A method of fixing the needle tube 2 to the needle holding member main body 5 will be described later.

The flange section 6 is composed of an annular member projecting radially outward, at an outer circumferential surface on the other end side of the needle holding member main body 5. The flange section 6 has two flat surfaces 6a and 6b formed to be perpendicular to the axial direction of the needle holding member main body 5. The flat surface 6a of the flange section 6 is formed to be substantially flush with an end face 5a on the other end side of the needle holding member main body 5.

The fitting section 7 is composed of a tubular member formed, at an outer edge of the flange section 6, to protrude from the flat surface 6a of the flange section 6 perpendicularly to the direction in which the proximal portion 2b of the needle tube 2 protrudes. The inside diameter of the fitting section 7 formed in a tubular shape is set to be slightly smaller than the outside diameter of a seal member fixing section 10, which will be described later. The outside diameter of the fitting section 7 is set to be approximately equal to or smaller than the outside diameter of a liquid containing member main body 9, which will be described later. In addition, the length of protrusion of the fitting section 7 from the flat surface 6a of the flange section 6 is set to be approximately equal to the length of protrusion of the seal member fixing section 10 (described later) from a bottom portion 9a of the liquid containing member main body 9.

The projected section 13 is formed to project in the axial direction of the needle holding member main body 5, in a central area of a flat surface region composed of the end face 5a on the other end side of the needle holding member main body 5 and the flat surface 6a of the flange section 6 which is continuous with the end face 5a. An end face of the projected section 13 is made to be a needle protrusion surface 13a from which the proximal portion 2b of the needle tube 2 protrudes. The projected section 13 is formed in such a shape as to be partly fitted into an upper portion of a fitting groove 10a of the seal member fixing section 10 (described later); specifically, it is formed in a truncated conical shape to be tapered off (decreased in diameter) toward the side of the proximal portion 2b of the needle tube 2. In addition, the axis of the projected section 13 coincides with the axis of the needle holding member main body 5.

Besides, in the needle holding member 3, a through-hole 14 to be penetrated by the needle tube 2 is provided along the axes of the needle holding member main body 5 and the projected section 13. A central portion of the needle tube 2 is fixed and held in the through-hole 14 by the high frequency welding. In this instance, the needle tube 2 is so fixed that its needle tip 2a capable of piercing a living body protrudes by a predetermined length from one end of the needle holding member main body 5 and its proximal portion 2b protrudes by a predetermined length from the needle protrusion surface 13a of the projected section 13.
Here, the length of protrusion of the needle tube 2 from the needle protrusion surface 13a is so set as not to protrude into the inside of the liquid chamber 12 of the liquid containing member 4 (described later), in an assembled state, as shown in FIG. 1.

The seal member 8 is composed of a truncated conical elastic member having a circular bottom face 8c and a circular top face 8b larger than the bottom face 8c in diameter. The seal member 8 is formed therein with an insertion hole 8a, in which the needle tube 2 is to be inserted and passed, along an axis passing through the centers of the top face 8b and the bottom face 8c thereof. The insertion hole 8a is formed to be smaller than the outside diameter of the needle tube 2. In addition, a surface of the seal member 8 is coated with a silicone oil, for facilitating assemblage.

The seal member 8 is held on the outer circumferential surface of the needle tube 2 so that a portion of the needle tube 2 which is on the proximal portion 2b side is inserted and passed in the insertion hole 8a, the top face 8b abuts on the projected section 13 of the needle holding member 3, and the proximal portion 2b of the needle tube 2 protrudes from the bottom face 8c, at the time of assemblage. In addition, the insertion hole 8a is formed to have a diameter smaller than the outside diameter of the needle tube 2; therefore, when the portion of the needle tube 2 which is on the proximal portion 2b side is inserted and passed in the insertion hole 8a, at the time of assemblage, the seal member 8 is held liquid-tight on the outer circumferential surface on a side of the proximal portion 2b of the needle tube 2 by an elastic force of its own.

As for a material of the seal member 8, the seal member 8 is formed of an elastic material for securing good air-tightness between the seal member 8 and the needle tube 2, and good air-tightness between the seal member 8 and the seal member fixing section 10 (described later). Examples of the elastic member include various rubber materials such as natural rubber, butyl rubber, isoprene rubber, butadiene rubber, styrene-butadiene rubber, silicone rubbers, and isobutylene rubber, various thermoplastic elastomers based on polyurethane, polyester, polyamide, olefin or styrene, and mixtures of them.

The liquid containing member 4 includes the liquid containing member main body 9, and the seal member fixing section 10 formed at one end of the liquid containing member main body 9. The liquid containing member main body 9 and the seal member fixing section 10 are formed integrally. As a material for the liquid containing member 4, materials which are the same as or similar to those for the needle holding member 3 as above-mentioned can be used. Incidentally, it is preferable for the material of the liquid containing member 4 to be substantially transparent, for securing inside visibility.

The liquid containing member main body 9 constitutes a syringe (outer tube) to be filled with a drug, in a prefilled syringe. The liquid containing member main body 9 is formed in a bottomed tubular shape provided with a bottom portion 9a at one end thereof and opened at the other end thereof (not shown). A space surrounded by the bottom portion 9a and a gasket (not shown) provided on the opened end side is made to be a liquid chamber 12 which is filled with the drug.

The outside diameter of the liquid containing member main body 9 is set to be approximately equal to the outside diameter of the fitting section 7 of the needle holding member 3. The bottom portion 9a of the liquid containing member main body 9 is formed in its center with a liquid passing hole 11 which penetrates through the bottom portion 9a. The liquid passing hole 11 is a hole in which the proximal portion 2b of the needle tube 2 is inserted and passed. The liquid passing hole 11 has a diameter slightly larger than the outside diameter of the proximal portion 2b of the needle tube 2. In the liquid passing hole 11, passage of liquid between the needle tube 2 and the liquid chamber 12 is conducted.

A plunger (not shown) is connected to the side, opposite to the side facing the liquid chamber 12, of the gasket. The plunger is protruding to the exterior via an opening at the other end of the liquid containing member main body 9. By operating the plunger, the gasket is moved in the axial direction inside the liquid containing member main body 9 and the drug filled in the liquid chamber 12 is discharged.

The seal member fixing section 10 is formed in a tubular shape to protrude in a perpendicular direction from a surface, on the side opposite to the side for facing the liquid chamber 12, of the bottom portion 9a of the liquid containing member main body 9. The seal member fixing section 10 is opened at its end portion (tip or distal end 10b) on the side opposite to the side of the liquid containing member main body 9. The inner circumferential surface of the seal member fixing section 10 is formed to be decreased in diameter, in a tapered form, from the tip 10b of the seal member fixing section 10 to the bottom portion 9a of the liquid containing member main body 9.

A recessed groove surrounded by the tapered inner circumferential surface of the seal member fixing section 10 and the bottom portion 9a of the liquid containing member main body 9 is configured as the fitting groove 10a to fit the seal member 8. In addition, the outside diameter of the seal member fixing section 10 is set to be smaller than the inside diameter of the fitting section 7 of the needle holding member 3. The length of protrusion of the seal member fixing section 10 from the bottom portion 9a of the liquid containing member main body 9 is set to be approximately equal to the length of protrusion of the fitting section 7 of the needle holding member 3.

In this embodiment, in an assembled state, as shown in FIG. 1, the seal member 8 attached to the proximal portion 2b of the needle tube 2 is fitted in the fitting groove 10a of the seal member fixing section 10 of the liquid containing member 4, and the proximal portion 2b of the needle tube 2b is inserted and passed in the liquid passing hole 11 in the liquid containing member main body 9. In this instance, the length of protrusion of the proximal portion 2b of the needle tube 2 from the needle protrusion surface 13a is so configured that the proximal portion 2b does not protrude into the inside of the liquid chamber 12, as above-mentioned; therefore, the proximal portion 2b is accommodated in the liquid passing hole 11.

The fitting section 7 of the needle holding member 3 is fitted onto and connected to the outer circumferential surface of the seal member fixing section 10 of the liquid containing member 4, whereby the needle holding member 3 and the liquid containing member 4 are connected to each other in a fixed state. In this instance, the length of protrusion of the seal member fixing section 10 from the bottom portion 9a of the liquid containing member main body 9 is set to be approximately equal to the length of protrusion of the fitting section 7 of the needle holding member 3. Therefore, the tip 10b of the seal member fixing section 10 is fixed in the state of abutting on the flat surface 6a of the flange section 6.

Here, in the medical instrument with attached needle 1 in this embodiment, as shown in FIG. 2, in a pre-assembly state, the minimum outside diameter W2 of the seal member 8 is greater than the minimum inside diameter W1 of the fitting groove 10a and smaller than the maximum inside diameter W3 of the fitting groove 10a. In addition, the maximum outside diameter W4 of the seal member 8 is set to be greater than the maximum inside diameter W3 of the fitting groove 10a. Further, the height H2 in the axial direction of the seal member 8 is set to be approximately equal to or greater than the depth H1 of the fitting groove 10a. Incidentally, in this embodiment, W1 = 4.4 mm, W2 = 4.8 mm, W3 = 4.8 mm, W4 = 5.2 mm, H1 = 3.0 mm, and H2 = 3.3 mm. Besides, the liquid passing hole 11 has a diameter W5 = 0.5 mm, and the liquid containing section 9 has an outside diameter W6 = 8 mm.

This ensures that in the case where the seal member 8 is inserted into the fitting groove 10a, the seal member 8 is fitted into the fitting groove 10a while being compressed, so that a pressure is exerted on the seal member 8 from the seal member fixing member 10. In addition, the projected section 13 of the needle holding member 3 is fixed by entering into an upper portion of the fitting groove 10a, whereby a pressure in the direction from the top face 8b toward the bottom face 8c of the seal member 8 is exerted. As a result, liquid-tightness is maintained between the seal member 8 and the needle tube 2 as well as between the seal member 8 and the liquid containing member 4. Thus, in this embodiment, the seal member 8 is compressed by and clamped between the needle holding member 3 and the liquid containing member 4, in the condition where the proximal portion 2b of the needle tube 2 and the liquid chamber are in communication with each other.

### [1-2. Method for Assembly of Medical Instrument with Attached Needle]

Now, a method for assembling the medical instrument with attached needle 1 configured as above will be described below.

First, in the needle holding member 3, the needle tip 2a capable of piercing a living body of the needle tube 2 is protruded from the one end of the needle holding member main body 5, and the proximal portion 2b of the needle tube 2 is protruded from the needle protrusion surface 13a of the projected section 13. In this condition, the middle portion of the needle tube 2 and the needle holding member main body 5 are fixed by high frequency welding. This results in that the needle 2 is fixed so as not to be detached from the needle holding member 3.

Next, the proximal portion 2b of the needle tube 2 is inserted and passed in the insertion hole 8a from the top face 8b side of the seal member 8, whereby the seal member 8 is held in close contact with the proximal portion 2b side of the needle tube 2. Then, the needle holding member 3 holding the needle tube 2 penetrating through the seal member 8 is moved toward the liquid containing member 4 side, while performing a position adjustment such that the proximal portion 2b of the needle tube 2 is inserted and passed in the liquid passing hole 11 of the liquid containing member 4.

This results in that the seal member 8 held on the side of the proximal portion 2b of the needle tube 2 is fitted into the fitting groove 10a of the seal member fixing section 10 while being compressed, and the fitting section 7 of the needle holding member 3 is fitted onto the seal member fixing section 10 of the liquid containing member 4. Then, the needle holding member 3 is moved until the tip 10b of the seal member fixing section 10 abuts on the flat surface 6a of the flange section 6, whereby fitting and connection of the needle holding member 3 and the liquid containing member 4 to each other is completed.

The fixing of the needle holding member 3 and the liquid containing member 4 at the time of fitting and connection of them to each other can be performed by a method in which the fitting section 7 of the needle holding member 3 is formed with a male screw at the inner circumference thereof, whereas the seal member fixing section 10 of the liquid containing member 4 is formed with a female screw at the outer circumference thereof, and the male and female screws are engaged with each other. Other than this, a method may also be adopted in which the fitting section 7 is provided at its inner circumference with a claw or claws capable of being locked on the outer circumference of the seal member fixing section 10, and locking is conducted. Further, the fixing of the needle holding member 3 and the liquid containing member 4 may be performed by use of an adhesive. For avoiding mixing of such an adhesive into the drug contained in the liquid chamber 12, however, it is preferable for the fixing to be realized by screw engagement or locking.

In the case where the needle holding member 3 and the liquid containing member 4 are fitted and connected to each other completely, as shown in FIG. 1, the needle protrusion surface 13a of the projected section 13 formed as a part of the needle holding member 3 abuts on the top face 8b of the seal member 8 and pushes in the seal member 8 toward the side of the liquid containing member 4, and the projected section 13 slightly comes into the fitting groove 10a. In this case, as above-mentioned, the height H2 from the top face 8b to the bottom face 8c of the seal member 8 in a pre-assembly state is set to be equal to or larger than the depth H1 of the fitting groove 10a. When the projected section 13 comes into the fitting groove 10a, therefore, the seal member 8 is compressed toward the side of the liquid containing member 4.

Meanwhile, since the seal member 8 is composed of the elastic member, an elastic force is generated at the time of assemblage. Since the needle holding member 3 and the liquid containing member 4 are fitted and connected to each other in a fixed state, however, the compressing force on the seal member 8 toward the side of the liquid containing member 4 is maintained. In an assembled state, the seal member 8 and the needle tube 2 are held onto each other in a liquid-tight manner, and the seal member 8 and the fitting groove 10a are held onto each other in a liquid-tight manner, by the elastic force generated in the seal member 8.

In addition, the outside diameter of the seal member 8 in the pre-assembly state is set to be generally a little larger than the inside diameter of the fitting groove 10a. In this case, however, the minimum outside diameter W2 of the seal member 8 is smaller than the maximum inside diameter W3 of the fitting groove 10a, and the seal member 8 and the fitting groove 10a are each formed in a tapered shape. Therefore, fitting of the seal member 8 into the fitting groove 10a can be carried out easily. Besides, since the surface of the seal member 8 is coated with a silicone oil, the fitting-in can be performed smoothly. Furthermore, since the seal member 8 can be fitted into the fitting groove 10a simultaneously when the needle holding member 3 is fitted and connected to the liquid containing member 4, the fitting of the seal member 8 into the fitting groove 10a can be carried out easily.

Then, after the needle holding member 3 and the liquid containing member 4 are fitted and connected to each other, as shown in FIG. 1, the liquid chamber 12 of the liquid containing member main body 9 is filled with a desired drug, followed by sealing with the gasket. As a result, the medical instrument with attached needle (prefilled syringe) 1 in this embodiment is completed.

Incidentally, the drug with which the medical instrument with attached needle 1 in this embodiment can be filled may be any drug that is ordinarily used as an injection. Examples of the drug include protein drugs such as antibodies, etc., peptide drugs such as hormones, etc., nucleic acid drugs, cell drugs, blood derivatives, vaccines for prevention of various infectious diseases, carcinostatic agents, anesthetics, narcotics, antibiotics, steroid preparations, proteinase inhibitors, heparin, saccharide injections such as glucose, etc., electrolyte correction injections such as sodium chloride, potassium lactate, etc., vitamin preparations, lipid emulsions, and contrast media.

In this embodiment, the needle holding member 3 for holding the needle tube 2 and the liquid containing member 4 for containing the drug therein are configured as separate members. This ensures that, even in the case where fixing of the needle tube 2 to the needle holding member 3 by the high frequency welding is accompanied by generation of continuous bubbles in the needle holding member main body 5 so that cracking would occur in the needle holding member main body 5 due to the bubbles, the crack would not produce an influence on the liquid containing member 4. In addition, since the needle tube 2 and the liquid containing member 4 are fixed in a liquid-tight manner by the seal member 8, the drug contained in the liquid chamber 12 would not lead to the exterior. Besides, in this embodiment, no adhesive is used for fixing of the needle holding member 3 and the needle tube 2, so that the drug filling the liquid containing member 4 would not make direct contact with an adhesive. Therefore, there is no possibility of bad influences being imposed on the drug due to mixing-in of the adhesive. Accordingly, the medical instrument with attached needle 1 can be used with enhanced reliability.

While the example in which the projected section 13 is provided has been adopted in this embodiment, the projected section 13 may not necessarily be provided, and an example without provision of the projected section 13 may be adopted. The height H2 in the axial direction of the seal member 8 has been configured to be greater than the depth H1 of the fitting groove 10a, in the pre-assembly state. This configuration ensures that, even where the projected section 13 is not provided, the seal member 8 can be pressed by the end face 5a on the other end side of the needle holding member main body 5, thereby to exert a pressure in the axial direction on the seal member 8.

In addition, while the configuration has been adopted in which the seal member 8 is formed along its axis with the insertion hole 8a composed of a hole having a diameter smaller than the outside diameter of the needle tube 2, a configuration may be adopted in which the insertion hole 8a is composed of a slit and the needle tube 2 is passed through the slit. Thus, the insertion hole 8a formed in the seal member 8 may have any configuration that permits the needle tube 2 to be inserted and passed therein and that can hold the needle tube 2 in a secure-contact manner. In the case where a slit is formed as the insertion hole 8a, the insertion and passage of the needle tube 2 is conducted by inserting the needle tube 2 after broadening the slit by pressing the seal member 8 from the outer circumference side, whereby the needle tube 2 can be easily inserted and passed through the seal member 8.

Besides, in this embodiment, the configuration has been adopted in which the proximal portion 2b of the needle tube 2 does not protrude into the liquid chamber 12 in the assembled state. This ensures that at the time of discharging the drug by moving the gasket within the liquid chamber 12, the gasket can be moved until the gasket comes into contact with the bottom portion 9a of the liquid containing member main body 9. Accordingly, in using the medical instrument with attached needle 1, it is possible to reduce the amount of the liquid left in the liquid chamber 12.

### [1-3. Modification]

While the present embodiment has been described by taking the prefilled syringe as the example of the medical instrument with attached needle 1, a configuration may be adopted in which a needle tube attached to a tip of a tube for permitting blood or a medical agent to flow therethrough is attached to the medical instrument with attached needle according to the present invention. FIG. 3 shows a sectional view of a medical instrument with attached needle 20 according to a modification of the first embodiment. In FIG. 3, the parts corresponding to those in FIG. 1 are denoted by the same reference symbols as used in FIG. 1, and overlapping descriptions of the parts will be omitted.

In the medical instrument with attached needle 20 in the modification, a liquid containing member 24 is composed of a liquid containing member main body 21 and a seal member fixing section 10. The liquid containing member main body 21 constitutes a connection section for connection with a tube 22 which is connected to an infusion pack (not shown) or the like.

The liquid containing member main body 21 is formed in a bottomed tubular shape, which is provided with a bottom portion 21a at one end thereof and is opened at the other end thereof. The tube 22 formed of an elastic material is securely fitted onto the outer circumference of the opened other end of the liquid containing member main body 21. Such a configuration is applicable to an indwelling needle and a blood collection needle.

In the medical instrument with attached needle 20 in the modification, it suffices for the axial length of the liquid containing member main body 21 to ensure the length that the tube 22 to be joined can be stably held on the outer circumferential surface of the liquid containing member main body 21.

As shown in this modification, also in the medical instrument with attached needle 20 having the needle tube 2 to be attached to the tip of the tube 22, the needle holding member 3 with the needle tube 2 fixed thereto by the high frequency welding and the liquid containing member 24 having the liquid chamber 12 are configured as separate members. Even if a crack is generated in the needle holding member 3, therefore, the crack would not have any influence on the liquid containing member 24, and liquid leakage would not be induced.

### <2. Second Embodiment: Medical Instrument with Attached Needle>

Now, a medical instrument with attached needle 30 according to a second embodiment of the present invention will be described below. FIG. 4 is a sectional view showing a configuration of the medical instrument with attached needle 30 according to this embodiment. The medical instrument with attached needle 30 in this embodiment shows an example which differs from the first embodiment especially in a configuration of a seal member 33. In FIG. 4, the parts corresponding to those in FIG. 1 are denoted by the same reference symbols as used in FIG. 1, and overlapping descriptions of the parts will be omitted. This embodiment, also, will be described by taking a prefilled syringe as an example of the medical instrument with attached needle 30.

As shown in FIG. 4, the medical instrument with attached needle 30 in this embodiment includes a needle tube 2, a needle holding member 31 for holding the needle tube 2, a seal member 33, and a liquid containing member 32 having a liquid chamber 12 capable of holding a drug therein.

The needle holding member 31 is configured to have a needle holding member main body 5, a flange section 6, a fitting section 7, and a projected section 35, which are formed integrally. As a material for the needle holding member 31, materials which are the same as or similar to those for the needle holding member 3 in the first embodiment can be used.

The projected section 35 is formed to project in the axial direction of the needle holding member main body 5, in a central area of a flat surface region composed of an end face 5a on the other end side of the needle holding member main body 5 and a flat surface 6a of the flange section 6 which is continuous with the end face 5a. The end face of the projected section 35 is made to be a needle protrusion surface 35a from which a proximal portion 2b of the needle tube 2 protrudes. The projected section 35 is formed in a cylindrical shape and such a shape as to be partly fitted into an upper portion of a fitting groove 34a of a seal member fixing section 34 which will be described later. The maximum outside diameter of the projected section 35 is set to be slightly smaller than the inside diameter of the fitting groove 34a of the seal member fixing section 34 described later. In addition, the axis of the projected section 35 coincides with the axis of the needle holding member main body 5.

Besides, in the needle holding member 31, a through-hole 14 to be penetrated by the needle tube 2 is provided along the axes of the needle holding member main body 5 and the projected section 35. A central portion of the needle tube 2 is fixed and held in the through-hole 14 by high frequency welding. In this instance, the needle tube 2 is so fixed that its needle tip 2a capable of piercing a living body protrudes by a predetermined length on the upper side of the needle holding member main body 5 and its proximal portion 2b protrudes by a predetermined length from the needle protrusion surface 35a of the projected section 35.

Here, the length of protrusion of the needle tube 2 from the needle protrusion surface 35a is so set as not to protrude into the inside of the liquid chamber 12 of the liquid containing member 32 (described later), in an assembled state, as shown in FIG. 4.

The seal member 33 is composed of an O-ring-shaped member, and a central hole in the seal member thus formed in the O-ring-like shape is made to be an insertion hole 33a in which the needle tube 2 is to be inserted and passed. The insertion hole 33a is configured to have a diameter smaller than the outside diameter of the needle tube 2, and a proximal portion 2b of the needle tube 2 is inserted and passed in the insertion hole 33a. Upon assemblage, the seal member 33 is penetrated by the needle tube 2 so that its top face 33b abuts on the projected section 35 of the needle holding member 31. Since the insertion hole 33a is smaller than the outside diameter of the needle tube 2, the seal member 33 is held air-tight on the outer circumferential surface of the proximal portion 2b of the needle tube 2 by an elastic force of the seal member 33. In addition, the surface of the seal member 33 is coated with a silicone oil, for facilitating assemblage.
As a material for the seal member 33, materials which are the same as or similar to those for the seal member 8 in the first embodiment can be used.

The liquid containing member 32 includes a liquid containing member main body 9 which constitutes a syringe, and a seal member fixing section 34 formed at one end of the liquid containing member main body 9. The liquid containing member main body 9 and the seal member fixing section 34 are formed integrally. As a material for the liquid containing member 32, materials which are the same as or similar to those for the liquid containing member 4 in the first embodiment can be used. Incidentally, it is preferable that the material of the liquid containing member 4 is substantially transparent, for securing inside visibility.

The seal member fixing section 34 is formed in a tubular shape projecting in a perpendicular direction from that surface of a bottom portion 9a of the liquid containing member main body 9 which is on the side opposite to the side for facing the liquid chamber 12. A recessed groove surrounded by the inner circumferential surface of the seal member fixing section 34 formed in the tubular shape and the bottom portion 9a of the liquid containing member main body 9 is configured as a fitting groove 34a to fit the seal member 33. The outside diameter of the seal member fixing section 34 is set to be a little smaller than the inside diameter of the fitting section 7 of the needle holding member 31. The length of protrusion of the seal member fixing section 34 from the bottom portion 9a is set to be approximately equal to the length of protrusion of the fitting section 7 of the needle holding member 31.

In this embodiment, the seal member 33 attached to the proximal portion 2b of the needle tube 2 is fitted in the fitting groove 34a in the seal member fixing section 34 of the liquid containing member 32, and the proximal portion 2b of the needle tube 2 is inserted and passed in a liquid passing hole 11 in the liquid containing member main body 9. In this instance, the length of protrusion of the proximal portion 2b of the needle tube 2 from the needle protrusion surface 35a is so configured as not to protrude into the liquid chamber 12, in an assembled state, so that the proximal portion 2b of the needle tube 2 is accommodated in the liquid passing hole 11. Besides, the fitting section 7 of the needle holding member 31 is fitted and connected to the outer circumferential surface of the seal member fixing section 34, whereby the needle holding member 31 and the liquid containing member 32 are fixed and connected to each other.

Here, in the medical instrument with attached needle 30 in this embodiment, the outside diameter of the seal member 33 is set to be greater than the inside diameter of the fitting groove 34a, in the condition before assembling the instrument by fitting and connecting the needle holding member 31 and the liquid containing member 32 to each other. In addition, the height in the axial direction of the seal member 33 is set to be approximately equal to or greater than the depth of the fitting groove 34a. This ensures that, where the seal member 33 is fitted in the fitting groove 34a, the seal member 33 is fitted in a compressed state in the fitting groove 34a, so that a pressure is exerted on the seal member 33 from the seal member fixing section 34. Besides, the projected section 35 of the needle holding member 31 is fixed by entering into an upper portion of the fitting groove 34a, whereby a pressure directed from the top face 33b toward a bottom face of the seal member 33 is exerted. As a result, liquid-tightness is maintained between the seal member 33 and the needle tube 2, and between the seal member 33 and the liquid containing member 32.

The medical instrument with attached needle 30 in this embodiment can be assembled by a method which is the same as or similar to the assembling method for the medical instrument with attached needle 1 in the first embodiment. After the needle holding member 31 and the liquid containing member 32 are fitted and connected to each other, as shown in FIG. 4, the liquid chamber 12 in the liquid containing member main body 9 is filled with a desired drug, followed by sealing with a gasket, whereby the medical instrument with attached needle (prefilled syringe) 30 in this embodiment is completed.

In this embodiment, the needle holding member 31 for holding the needle tube 2 and the liquid containing member 32 for containing the drug therein are configured as separate members. This ensures that, even where fixing of the needle tube 2 and the needle holding member 31 to each other by the high frequency welding is accompanied by generation of continuous bubbles in the needle holding member main body 5 and a crack is generated in the needle holding member main body 5 due to the bubbles, the crack would not produce any influence on the liquid containing member 32. In addition, since the needle tube 2 and the liquid containing member 32 are fixed to each other in a liquid-tight manner by the seal member 33, the drug contained in the liquid chamber 12 would not leak to the exterior.

Besides, in this embodiment, no adhesive is used for fixing the needle holding member 31 and the needle tube 2; therefore, there is no risk that the drug contained in the liquid containing member 32 might make direct contact with an adhesive, thereby suffering no bad influence to the drug. This permits the medical instrument with attached needle 30 to be used with enhanced reliability. In addition, it is possible to obtain an effect which is the same as or similar to the effect obtained in the first embodiment.

Like in the modification of the first embodiment, the medical instrument with attached needle 30 in this embodiment is applicable to any medical instrument with attached needle that is used in the state of being attached to a tip of a tube permitting a medical agent or blood to flow therethrough.

Some embodiments of the medical instrument with attached needle according to the present invention have been described above, together with the operation and effect thereof. However, the medical instrument with attached needle of the present invention is not restricted to the above-described embodiments, and various modifications on a carrying-out basis are possible within the scope of the invention as defined by the claims.

### Industrial Applicability

The present invention is applicable to injectors, indwelling needles, and other medical instruments with attached needle.

### Explanation of Reference Symbols

1, 20, 30 ... Medical instrument with attached needle, 2 ... Needle tube, 3, 31 ... Needle holding member, 4, 24, 32 ... Liquid containing member, 5 ... Needle holding member main body, 6 ... Flange section, 7 ... Fitting section, 8, 33 ... Seal member, 8a, 33a ... Insertion hole, 9, 21 ... Liquid containing member main body, 10 ... Seal member fixing section, 10a, 34a ... Fitting groove, 10b ... Tip, 11 ... Liquid passing hole, 12 ... Liquid chamber, 13, 35 ... Projected section, 13a ... Needle protrusion surface, 14 ... Through-hole, 22 ... Tube

## Claims

1. A medical instrument with attached needle, comprising:
a needle tube having a needle tip capable of piercing a living body;
a needle holding member configured to fix and hold a middle portion of the needle tube by high frequency welding in a condition where the needle tip protrudes from one end of the needle holding member and a proximal portion on a side opposite to the needle tip of the needle tube protrudes from the other end of the needle holding member;
a seal member composed of an elastic member and provided on an outer circumference on a side of the proximal portion of the needle tube so as to be liquid-tight; and
a liquid containing member having a liquid chamber capable of containing a liquid therein,
wherein the seal member is compressed by and clamped between the needle holding member and the liquid containing member in a condition where the proximal portion of the needle tube and the liquid chamber communicate with each other.

2. The medical instrument with attached needle according to claim 1,
wherein the liquid containing member is provided with a fitting groove for compressing and holding the seal member at one end of the liquid containing member, an outside diameter of the seal member in a state before fitting into the fitting groove is greater than an inside diameter of the fitting groove, and a height of the seal member is greater than a depth of the fitting groove.

3. The medical instrument with attached needle according to claim 1,
wherein the needle holding member is provided with a projected section to be fitted into the fitting groove on a side of the other end of the needle holding member, and the projected section presses the seal member in a condition where the seal member is compressed and held in the fitting groove.

4. The medical instrument with attached needle according to claim 1,
wherein an inner circumferential surface of the fitting groove is formed in a tapered shape decreased in diameter along a depth direction of the fitting groove.

5. The medical instrument with attached needle according to claim 4,
wherein the seal member is formed in a truncated conical shape.

6. The medical instrument with attached needle according to claim 1,
wherein the seal member is composed of an O-ring.

7. The medical instrument with attached needle according to claim 1,
wherein the liquid containing member is composed of a syringe prefilled with a drug.
